# EUROPEAN PATENT APPLICATION

(11) **EP 4 292 625 A1**
(43) Date of publication of application: **20.12.2023**
(21) Application number: 22752119.2
(22) Date of filing: 24.01.2022
(51) Int. Cl.: A61M 5/20

(54) **INJECTION PUMP CONTROL METHOD, SYSTEM, AND INJECTION PUMP**

(30) Priority: 09.02.2021 CN 202110176360
(71) Applicant: Hangzhou Broncus Medical Co., Ltd., Hangzhou, Zhejiang 310051 (CN)
(72) Inventor: XU, Hong, Hangzhou, Zhejiang 310051 (CN); LI, Yuhang, Hangzhou, Zhejiang 310051 (CN); SUI, Hailong, Hangzhou, Zhejiang 310051 (CN); JIANG, Lifang, Hangzhou, Zhejiang 310051 (CN)
(74) Representative: Petraz, Gilberto Luigi
(86) International application number: PCT/CN2022/073574
(87) International publication number: WO 2022/170957

(57) **Abstract**

An injection pump control method, a system (100), and an injection pump (200). The injection pump control method comprises: when a control switch (41) is detected as being connected, a monitoring controller (11) in a dual controller unit (10) turns on a power source (21) to start supplying power; a master controller (12) in the dual controller unit (10) transmits a communication signal to the monitoring controller (11), controls a data collecting unit (30) to collect operational data of the injection pump (200), determines, on the basis of the operational data, whether the injection pump (200) is operating normally, and if an abnormality is found, then controls an alarm module (50) of the injection pump (200) to issue an alarm; and the monitoring controller (11) continues to acquire the communication signal transmitted by the master controller (12), when the communication signal is abnormal, cuts off the supply of power of the power source (21) and transmits an alarm signal, the cutoff of the supply of power triggering an emergency source (22) to be turned on to supply power to the alarm module (50), and the alarm module (50) issuing an alarm according to the alarm signal, thus mitigating the impact on the fault handling capability of the injection pump (200) when the master controller (12) malfunctions, and increasing operational safety.

## Description

### TECHNICAL FIELD

Embodiments of the present invention relate to the field of electronic technology, and in particular to an injection pump control method, system, and injection pump.

### BACKGROUND

Radio frequency (RF) technology is to transmit RF energy to a target area accurately under image guidance to perform ablation on a target object to be ablated. During an ablation process, an injection pump will cooperate with an ablation device to inject saline into the ablation site.

In the prior art, a control system of the injection pump cannot continue to control the injection pump when a controller of the injection pump broke down, which can only be manually handled after the operator discovers it, which is inefficient and may easily cause further damage to the injection pump and cause safety issues.

### SUMMARY OF THE INVENTION

### TECHNICAL PROBLEM

Embodiments of the present invention provides an injection pump control method, system, and injection pump, which can be used to continue controlling the injection pump when a master controller broke down by setting up another monitoring controller for the master controller, thereby improving an efficiency of fault handling, avoiding further damage to the injection pump, and improving safety.

### SOLUTION TO THE PROBLEM

### TECHNICAL SOLUTION

On one aspect, an embodiment of the present invention provides an injection pump control method that includes:

when a control switch is detected as being connected, a monitoring controller of a dual controller unit turning on a power source to start supplying power; a master controller of the dual controller unit transmitting communication signals to the monitoring controller, and controlling a data acquisition unit to collect operational data of an injection pump and determining whether the injection pump is operating normally according to the operational data, and controlling an alarm module of the injection pump to alarm when an abnormality is found; the monitoring controller continuously obtaining communication signals transmitted by the master controller, and cutting off the supply of power of the power source and transmitting an alarm signal when the communication signal is abnormal, the cutoff of the supply of power triggering an emergency source to be turned on to supply power to the alarm module, and the alarm module alarming according to the alarm signal.

On another aspect, an embodiment of the present invention further provides an injection pump control system that includes:
a dual controller unit, a dual power source unit, a data acquisition unit, a button control unit and alarm module; wherein the dual controller unit comprises a monitoring controller and a master controller, and the dual power source unit comprises a power source and an emergency source; the monitoring controller is connected to the master controller, the power source, the button control unit and the alarm module, respectively; the master controller is connected to the data acquisition unit, the emergency source and the alarm module, respectively; the emergency source is connected to the power source and the alarm module, respectively; the button control unit comprises a control switch, and the monitoring controller is connected to the control switch, the monitoring controller turning on the power source to start supplying power when the control switch is connected; the master controller is configured to transmit communication signals to the monitoring controller, and controls the data acquisition unit to collect operational data of the injection pump and determines whether the injection pump is operating normally according to the operational data, and controls the alarm module to alarm when an abnormality is found; the monitoring controller continuously obtains the communication signals transmitted by the master controller, and cuts off the supply of power of the power source and transmits an alarm signal when the communication signal is abnormal, wherein the cutoff of the supply of power triggers the emergency source to be turned on to supply power to the alarm module, and the alarm module alarms according to the alarm signal.

On another aspect, an embodiment of the present invention further provides an injection pump including an injection pump control system as described above.

### BENEFICIAL EFFECTS OF THE INVENTION

### BENEFICIAL EFFECTS

From the above embodiments of the present invention, it can be seen that by means of setting dual controllers and dual power supplies in the injection pump control system, the master controller is used to transmit communication signals to the monitoring controller, controls the data acquisition unit to collect operational data of the injection pump and determines whether the injection pump is operating normally according to the operational data, and controls the alarm module to alarm when an abnormality is found, thereby maintaining the normal working state of the injection pump independently; the monitoring controller is used to monitor whether there is a malfunction in the master controller through communication signals with the master controller, and cuts off the supply of power of the power source to the injection pump when there is a malfunction, triggers the emergency source to be turned on to supply power to the alarm module, and controls the alarm module to alarm at the same time, thereby preventing the possibility of further damage to the injection pump system in a control system with single controller which is failure to alarm in a timely manner as the whole pump control system is unable to respond due to a malfunction of the controller, achieving timely power outage and alarm when the master controller malfunctions, improving the efficiency of responding to the malfunction, and improving the operational safety of the injection pump.

### BRIEF DESCRIPTION OF THE DRAWINGS

### DESCRIPTION OF THE DRAWINGS

In order to describe the technical solutions according to the embodiments of the present invention or in the prior art more clearly, the drawings needed to be used in the embodiments or in the prior art will be described briefly below. Apparently, the drawings in the following description show some embodiments of the present invention. Other drawings can be obtained by persons of ordinary skill in the art according to these drawings without creative efforts.
FIG. 1 is a schematic view of an injection pump provided by an embodiment of the present invention.
FIG. 2 is a schematic view of an injection pump control system provided by an embodiment of the present invention.
FIG. 3 is a schematic view of a connection of an injection pump control system provided by another embodiment of the present invention to other modules of the injection pump.
FIG. 4 is a schematic view of a circuit connection of the injection pump control system provided by an embodiment of the present invention.
FIG. 5 is a flowchart of a control method of the injection pump control system provided by another embodiment of the present invention.
FIG. 6 is a flowchart of a control method of the injection pump control system provided by another embodiment of the present invention.
FIG. 7 is a schematic view of an injection pump provided by another embodiment of the present invention.

### EMBODIMENTS OF THE INVENTION

### DESCRIPTION OF THE EMBODIMENTS

In order to make the objects, technical solutions and advantages of the embodiments of the present invention clearer, the technical solutions according to the embodiments of the present invention will be clearly and completely described with reference to drawings in the embodiments of the present invention. Apparently, the embodiments described are merely some embodiments, but not all of the embodiments of the present invention. All other embodiments obtained by ordinary persons skilled in the art according to the embodiments of the present invention without creative efforts shall fall within the protection scope of the present invention.

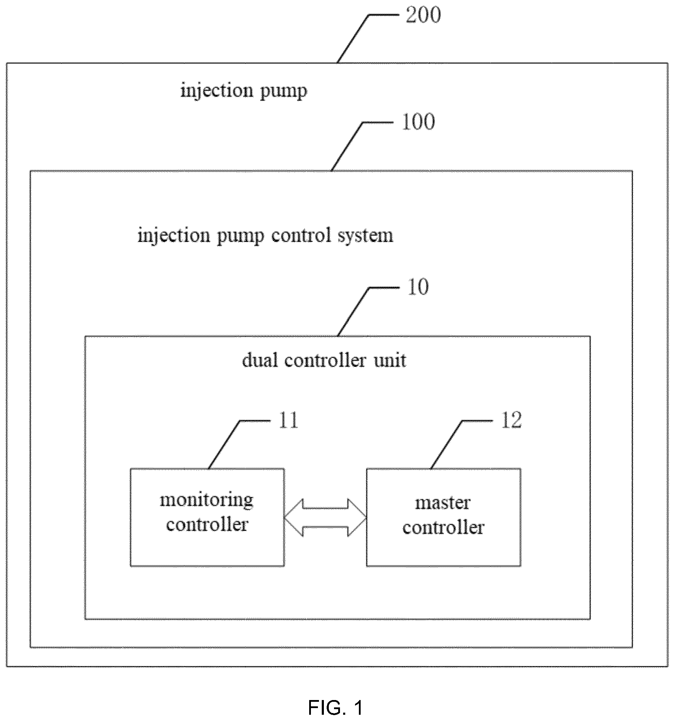

Referring to FIG. 1, which is a schematic view of an injection pump provided by an embodiment of the present invention. An injection pump control system 100 is provided in the injection pump 200, for controlling an operation of the injection pump 200. The injection pump control system 100 is a dual controller system, and includes a dual controller unit 10. The dual controller unit 10 includes a monitoring controller 11 and a master controller 12 which are connected together and both are MCUs (Micro controller Units). A specific model of the monitoring controller 11 may be STM32, and a specific model of the master controller 12 may be STM32F407ZGT6. The monitoring controller 11 is further connected to a switch button and a power source. When the switch button is connected, the injection pump starts, and the monitoring controller 11 controls the power source to start supplying power to the injection pump. The monitoring controller 11 may actively obtain or passively receive communication signals from the master controller 12. A function of the communication signals is to determine whether the monitoring controller 11 and the master controller 12 maintain normal communication according to the content sent and replied by them. Specifically, the communication signals may be heartbeat signals. When the communication signal sent by the master controller 12 is abnormal, it means that the master controller 12 may be unable to continue working due to malfunction. The monitoring controller 11 firstly turns off a motor of the injection pump, and then turns off the injection pump. That is, cutting off the power source of the injection pump, triggering an emergency source to be turned on to supply power to an alarm module, and then controlling the alarm module to alarm.

Furthermore, when the injection pump starts working, the master controller 12 samples operational data of the injection pump, specifically samples operational data of the motor, screen, buttons, sensors, slot-type optocouplers, limit switches, sound-light alarm modules, storage devices and external power supplies of the injection pump, and determines whether there are any abnormalities in the sampling data that trigger the alarm. If there are any abnormalities, the alarm module will be triggered to issue an alarm. The alarm triggered by abnormal sampling data and the alarm triggered by the malfunction of the master controller 12 are in different manners. By comparison, the alarm triggered by the malfunction of the master controller 12 has a higher alertness, which may be, for example, an alarm light with the highest flashing frequency and red color, an alarm sound with the highest volume, and accompanied by flashing red letters on the screen of the injection pump.

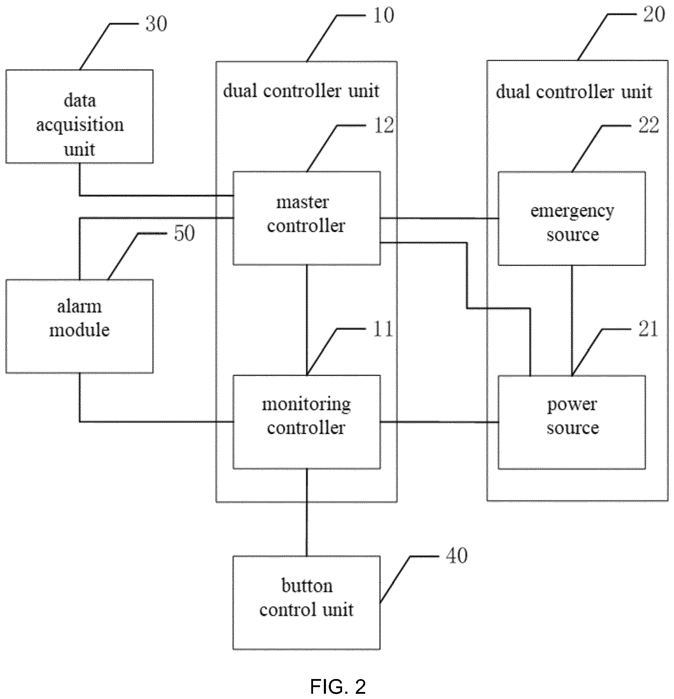

Referring to FIG. 2, which is a schematic view of an injection pump control system provided by an embodiment of the present invention. An injection pump system includes the injection pump control system which includes a dual controller unit 10, a dual power source unit 20, a data acquisition unit 30, a button control unit 40 and an alarm module 50, wherein
the dual controller unit 10 includes a monitoring controller 11 and a master controller 12;
the dual power source unit 20 includes a power source 21 and an emergency source 12;
the monitoring controller 11 is connected to the master controller 12, as well as the power source 21, the button control unit 40 and the alarm module 50 of the injection pump;
the master controller 12 is connected to the data acquisition unit 30, the emergency source 12 and the alarm module 50;
the emergency source 12 is further connected to the power source 21 and the alarm module 50, and starts to replace the power source 21 to start supplying power to the alarm module 50 when detecting that the power source 21 stops supplying power;
the button control unit 40 includes a control switch 41 connected to the monitoring controller 11 which starts the power source 21 to start supplying power to the injection pump (including the monitoring controller 11 and the master controller 12) when the control switch 41 is connected.

The master controller 12 is used to transmit communication signals to the monitoring controller 11, and controls the data acquisition unit 30 to collect operational data of the injection pump and determines whether the injection pump is operating normally according to the operational data, and controls the alarm module 50 to alarm when an abnormality is found. The operational data includes the operational data of the motor, power source, emergency source, display screen, injection device and various buttons of the injection pump, which may indicate whether the injection pump is in normal operation status.

The monitoring controller 11 is used to obtain the communication signals from the master controller 12 continuously, and confirms that the master controller 12 has a preset malfunction when the communication signal is abnormal, thereby cutting off the power source 21 of the injection pump and issuing an alarm signal. Cutting off the supply of power may trigger the emergency source 12 to start supplying power to the alarm module 50, so that the alarm module 50 alarms based on the alarm signal. At this time, the alarm is the highest level alarm, with the strongest prompting effect. The highest level alarm includes an alarm with the highest volume, an alarm with the highest frequency of buzzing and vibration, an alarm with flashing red letters and the longest duration.

The communication signal is obtained by the monitoring controller 11 from the master controller 12 or actively transmits by the master controller 12 to the monitoring controller 11 every preset time. The monitoring controller 11 determines whether the master controller 12 is malfunctioned based on the time and content of the obtained communication signal.

The injection pump control system described above may have another connection manner: the monitoring controller 11 is connected to the master controller 12, as well as the power source 21, the emergency source 12 and the alarm module 50 of the injection pump;
the monitoring controller 11 obtains the communication signal from the master controller 12, and cuts off the power source 21 of the injection pump and activates the emergency source 12 to start supplying power to the alarm module 50 when the communication signal is abnormal. The monitoring controller 11 is also used to control the alarm module 50 to alarm.

In this embodiment, by means of setting dual controllers and dual power supplies in the injection pump control system, the master controller is used to transmit communication signals to the monitoring controller, controls the data acquisition unit to collect operational data of the injection pump and determines whether the injection pump is operating normally according to the operational data, and controls the alarm module to alarm when an abnormality is found, thereby maintaining the normal working state of the injection pump independently; the monitoring controller is used to monitor whether there is a malfunction in the master controller through communication signals with the master controller, and cuts off the supply of power of the power source to the injection pump when there is a malfunction, triggers the emergency source to be turned on to supply power to the alarm module, and controls the alarm module to alarm at the same time, thereby preventing the possibility of further damage to the injection pump system in a control system with single controller which is failure to alarm in a timely manner as the whole pump control system is unable to respond due to a malfunction of the controller, achieving timely power outage and alarm when the master controller malfunctions, improving the efficiency of responding to the malfunction, and improving the operational safety of the injection pump.

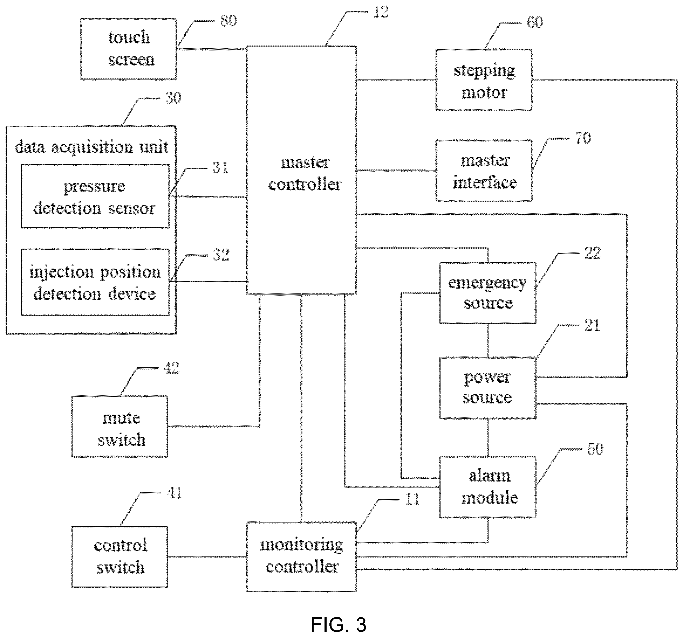

Furthermore, referring to FIG. 3, it shows a construction of an injection pump control system provided by another embodiment and a connection of the injection pump control system to other modules of the injection pump.

Furthermore, the injection pump further includes a motor, specifically a stepping motor 60. The monitoring controller 11 and the master controller 12 are respectively connected to the motor 60 of the injection pump. The monitoring controller 11 is used to control the motor 60 to stop running according to a preset gradual-stop mode when the communication signal is abnormal. This gradual-stop mode sets the required time and speed reduction rules for the motor 60 to slow down to 0, stopping the operation of the motor 60 firstly, and then turning off the supply of power of the power source 13.

Furthermore, the master controller 12 is connected to the motor 60 to control the push and pull of a handle of an injector, which in turn pushes and pulls an injection plunger of the injector to complete the injection operation.

Furthermore, the master controller 12 is further connected to a master interface 70 of the injection pump. An RF host, such as an RF ablation apparatus, is connected to the injection pump, and controls the injection pump to complete an injection task. When it is detected that the master interface 70 is connected to the RF host through a master control line, the master controller 12 is used to control the injection pump to enter a slave device mode, that is, controls inputting equipment of the injection pump to stop responding to input operations, wherein the inputting equipment includes a touch screen, a mouse, a keyboard, and etc.

The button control unit 40 further includes a mute switch 42, and the master controller 12 is further connected to the mute switch 42. When the mute switch 42 is connected, the master controller 12 detects a current output sound signal and determines which module is outputting the sound signal. According to the features of the module, choose a manner for cutting off the output of the sound signal, such as cutting off an output end of the sound generating structure, or cutting off a signal input of an input end of the module and cutting off the output of the sound signal. The sound signal, for example may be an alarm beep or alarm sound of the alarm module 50.

Furthermore, the injection pump further includes a touch screen 80, through which setting data for the injection pump can be obtained.

Furthermore, the data acquisition unit 30 includes a pressure detection sensor 31 and an injection position detection device 32.

The pressure detection sensor 31 is provided in a handle of an injector to measure a pressure of the stepping motor 60 on the handle, wherein the handle can push an injection plunger of the injector. By detecting the pressure, the pressure of the stepping motor 60 on the handle can be adjusted, thereby achieving a control of an injection flow rate or an injection volume.

The injection position detection device 32 includes an optocoupler and an optical baffle. The optical baffle is driven by the stepper motor 60 to move in a straight line, along with the plunger of the injector. Through a cooperation of the optical baffle and the optocoupler, a position of the plunger of the injector may be determined, and further a remaining injection volume and other working state data of the injector may be calculated based on a preset algorithm.

The master controller 12 acquires the working status data of the stepping motor 60, the pressure detection sensor 31, the injection position detection device 32 and the touch screen 80, and determines whether the corresponding module or device works normally according to these data. If the data abnormal, the alarm module 50 will be triggered according to a preset processing logic for alarming. An alarm manner of the alarm module 50 is one or any combinations of sound, light, vibration, and etc.

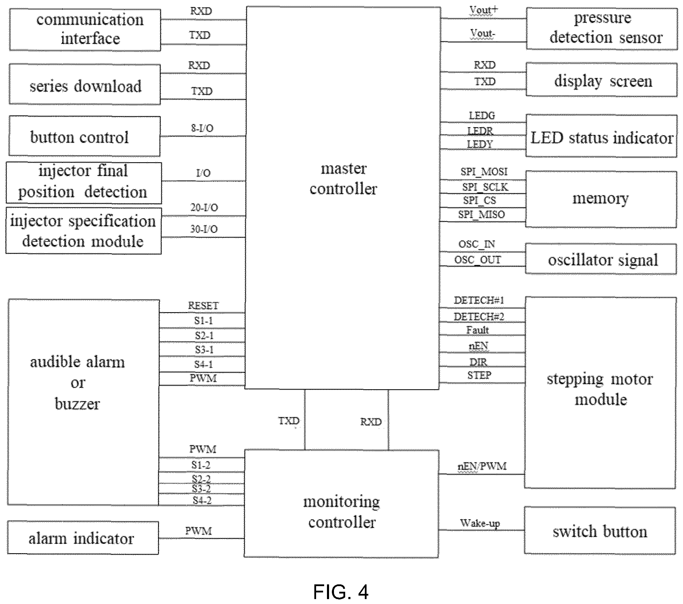

Referring to FIG. 4, which shows a circuit connection of some modules, including the monitoring controller and master controller, of the injection pump. As shown in FIG. 4, the monitoring controller is connected to the master controller, an audible alarm/buzzer module, an alarm indicator(LED light), a switch button and etc. The master controller is further connected to a plunger position detection module of the injector, an injection specification detection module, a communication interface, a memory, a display screen, an LED status indicator module for indicating a working status of the injection pump, etc. A connection manner of a receiving circuit is shown in the drawings, and a labeling of the connection manner is a general abbreviation in the field of electronic circuits and communication, for example, RXD represents receive data, TXD represents transmit data, I/O represents input/output, etc., which will not be repeated here.

In this embodiment, by means of setting dual controllers in the injection pump control system, the monitoring controller is used to monitor whether there is a malfunction in the master controller through communication signals with the master controller. If there is a malfunction, the monitoring controller cuts off the supply of power of the power source to the injection pump and triggers the emergency source to be turned on to supply power to the alarm module, and controls the alarm module to alarm at the same time, thereby preventing the possibility of further damage to the injection pump system in a control system with single controller which is failure to alarm in a timely manner as the whole pump control system is unable to respond due to a malfunction of the controller, achieving timely power outage and alarm when the master controller malfunctions, improving the efficiency of responding to the malfunction, and improving the operational safety of the injection pump. Since the monitoring controller is only responsible for monitoring the simple and important functions of the master controller, such as malfunctions, switching of the power supplies, and alarms, the possibility of malfunction of the monitoring controller itself may be reduced. At the same time, the master controller samples the operational data of the injection pump system to timely detect other malfunctions in the system, and cooperates with the monitoring controller to improve the fault tolerance of the injection pump control system, reducing the possibility of failure to respond in a timely manner when a malfunction occurs.

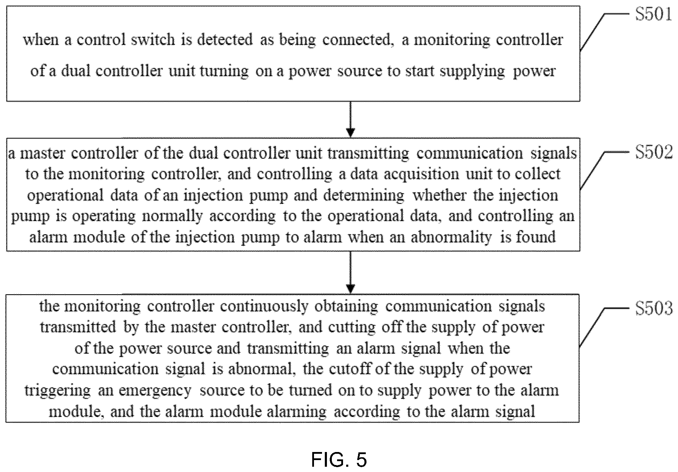

Referring to FIG. 5, which shows a flowchart of a control method of the injection pump control system provided by an embodiment of the present invention. As shown in FIG. 5, the control method of the injection pump control system may be applied to the injection pump, and an executing body may be a dual controller unit of the injection pump control system. A construction of the injection pump control system may refer to the above embodiments shown in FIG. 2 to FIG. 4. The method specifically includes:

Step S501: when a control switch is detected as being connected, a monitoring controller of a dual controller unit turning on a power source to start supplying power;
wherein the dual controller unit includes the monitoring controller and a master controller.

When the control switch of the injection pump is connected, the monitoring controller turns on the power source to start supplying power to the injection pump.

Step S502: the master controller of the dual controller unit transmitting communication signals to the monitoring controller, and controlling a data acquisition unit to collect operational data of an injection pump and determining whether the injection pump is operating normally according to the operational data, and controlling an alarm module of the injection pump to alarm when an abnormality is found;

the master controller transmits the communication signals to the monitoring controller according to a preset duration, wherein the communication signals may be heartbeat signals. This enables the monitoring controller to determine that a preset malfunction is generated in the master controller when the communication signal is abnormal. The preset malfunction includes serious malfunctions such as a short circuit in the master controller. The monitoring controller then cuts off the working motor and further cuts off the power source of the injection pump, triggers the emergency source to be turned on to supply power to the alarm module, and then triggers the alarm module to alarm according to a preset manner.

Further, the master controller controls the data acquisition unit to collect operational data of the injection pump and determines whether the injection pump is operating normally according to the operational data. If there are any abnormalities, the master controller controls the alarm module of the injection pump to issue an alarm. Whether the working state of the injection pump is normal may be confirmed by comparing the operational data with a preset standard data in the system.

Step S503: the monitoring controller continuously obtaining communication signals transmitted by the master controller, and cutting off the supply of power of the power source and transmitting an alarm signal when the communication signal is abnormal, the cutoff of the supply of power triggering an emergency source to be turned on to supply power to the alarm module, and the alarm module alarming according to the alarm signal.

The monitoring controller obtains the heartbeat signals transmitted by the master controller continuously. When the heartbeat signal is abnormal, such as not receiving the heartbeat signal from the master controller at the preset time and frequency, or a data packet in the heartbeat signal being abnormal, it indicates that the master controller has a preset malfunction that affects the operation of the injection pump and the operation of the injection pump must be stopped immediately.

The preset malfunctions include malfunctions that prevent the master controller from working continuously, such as short circuits in the internal circuit of the master controller, circuit faults in the master controller, etc.

In addition to connecting the master controller, the monitoring controller is further connected to the motor, the power source, the temporary power source and the alarm module of the injection pump, wherein the alarm module includes a sound alarm module and a light alarm module, and the power source is an external power source.

When the monitoring controller determines that there is a preset malfunction in the master controller, it promptly cuts off the supply of power of the power source of the injection pump and sends the alarm signal. Cutting off the supply of power of the power source may trigger the emergency source to be turned on to supply power to the alarm module, which will alarm according to the alarm signal.

In this embodiment, by means of setting dual controllers and dual power supplies in the injection pump control system, the master controller is used to transmit communication signals to the monitoring controller, and controls the data acquisition unit to collect operational data of the injection pump and determines whether the injection pump is operating normally according to the operational data, and controls the alarm module to alarm when an abnormality is found, thereby maintaining the normal working state of the injection pump independently; the monitoring controller is used to monitor whether there is a malfunction in the master controller through communication signals with the master controller, and cuts off the supply of power of the power source to the injection pump when there is a malfunction, triggers the emergency source to be turned on to supply power to the alarm module, and controls the alarm module to alarm at the same time, thereby preventing the possibility of further damage to the injection pump system in a control system with single controller which is failure to alarm in a timely manner as the whole pump control system is unable to respond due to a malfunction of the controller, achieving timely power outage and alarm when the master controller malfunctions, improving the efficiency of responding to the malfunction, and improving the operational safety of the injection pump.

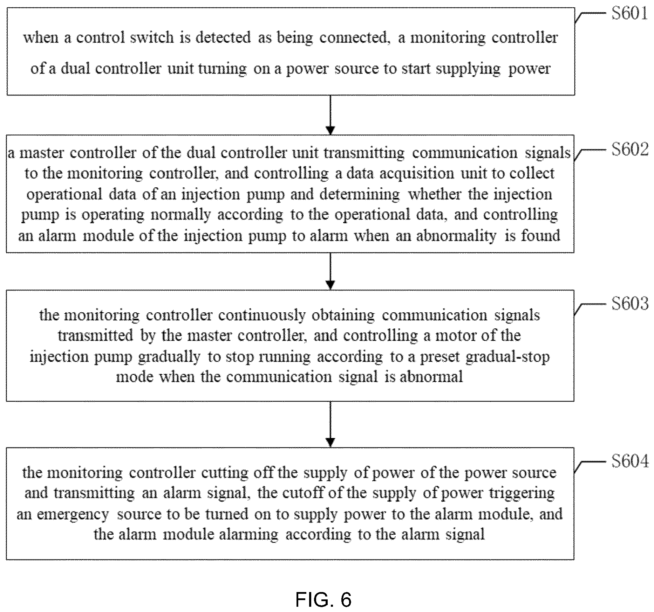

Referring to FIG. 6, which is a flowchart of the control method of the injection pump control system provided by another embodiment of the present invention. The control method of the injection pump control system may be applied to the injection pump control system, and includes:
Step S601: when a control switch is detected as being connected, a monitoring controller of a dual controller unit turning on a power source to start supplying power;
   wherein the dual controller unit includes the monitoring controller and a master controller.
   when the control switch of the injection pump is connected, the monitoring controller activates the power source to supply power to the injection pump.
Step S602: the master controller of the dual controller unit transmitting communication signals to the monitoring controller, and controlling a data acquisition unit to collect operational data of an injection pump and determining whether the injection pump is operating normally according to the operational data, and controlling an alarm module of the injection pump to alarm when an abnormality is found;
   the master controller transmits the communication signals to the monitoring controller according to a preset duration, wherein the communication signals may be heartbeat signals. This enables the monitoring controller to determine that a preset malfunction is generated in the master controller when the communication signal is abnormal. The preset malfunction includes serious malfunctions such as a short circuit in the master controller. The monitoring controller then cuts off the working motor and further cuts off the power source of the injection pump, triggers the emergency source to be turned on to supply power to the alarm module, and then triggers the alarm module to alarm according to a preset manner. In this time, the alarm prompts lower than the alarm when the master controller generates a preset malfunction, which may be, for example flashing yellow letters, low volume, low-frequency flashing, etc.

Further, the master controller controls the data acquisition unit to collect operational data of the injection pump and determines whether the injection pump is operating normally according to the operational data. If there are any abnormalities, the master controller controls the alarm module of the injection pump to alarm. Whether the working state of the injection pump is normal may be confirmed by comparing the operational data with a preset standard data in the system.

Specifically, the operational data includes the operational data of the motor, power source, emergency source, display screen, injection device and various buttons of the injection pump, which may indicate an operation status of the injection pump.

Step S603: the monitoring controller continuously obtaining communication signals transmitted by the master controller, and controlling a motor of the injection pump gradually to stop running according to a preset gradual-stop mode when the communication signal is abnormal;
when the heartbeat signal is abnormal, such as not receiving the heartbeat signal from the master controller at the preset time and frequency, or a data packet in the heartbeat signal being abnormal, it indicates that the master controller has a preset malfunction that affects the operation of the injection pump, and it is necessary to stop the operation of the injection pump. Firstly, the motor is controlled to gradually stop running to prevent damage to the machine caused by a sudden stop.

Step S604: the monitoring controller cutting off the supply of power of the power source and transmitting an alarm signal, the cutoff of the supply of power triggering an emergency source to be turned on to supply power to the alarm module, and the alarm module alarming according to the alarm signal.

Furthermore, when it is detected that a master control line of the injection pump is connected to a RF host, the master controller controls inputting equipment of the injection pump to stop responding to input operations and execute instructions issued by the RF host. When the master controller detects that the master control line of the injection pump is connected to the RF host, it controls the injection pump to enter the slave device mode, that is, controls the inputting equipment of the injection pump, including the keyboard, mouse, touch screen, etc., to stop responding to input operations. All operation instructions are input by the RF host as the master, and the injection pump is controlled by the operation instructions of the RF host. Specifically, the RF host may be an ablation apparatus.

Furthermore, when the mute switch 42 is detected to be pressed, the master controller detects a current output sound signal and correspondingly cuts off the output sound signal. For example, when the alarm module performs an audible and visual alarm and the master controller detects that the mute button is pressed, the alarm module is prohibited from outputting an audible signal.

For other details not described in this embodiment, please refer to the description of the embodiments shown in FIG. 2 to FIG. 5.

The control method of the injection pump control system provided by the embodiment of the present invention, by means of setting dual controllers in the injection pump control system, the monitoring controller is used to monitor whether there is a malfunction in the master controller through communication signals with the master controller. If there is a malfunction, the monitoring controller cuts off the supply of power of the power source to the injection pump and triggers the emergency source to be turned on to supply power to the alarm module, and controls the alarm module to alarm at the same time, thereby preventing the possibility of further damage to the injection pump system in a control system with single controller which is failure to alarm in a timely manner as the whole pump control system is unable to respond due to a malfunction of the controller, achieving timely power outage and alarm when the master controller malfunctions, improving the efficiency of responding to the malfunction, and improving the operational safety of the injection pump. Since the monitoring controller is only responsible for monitoring the simple and important functions of the master controller, such as malfunctions, switching of the power supplies, and alarms, the possibility of malfunction of the monitoring controller itself may be reduced. At the same time, the master controller samples the operational data of the injection pump system to timely detect other malfunctions in the system, and cooperates with the monitoring controller to improve the fault tolerance of the injection pump control system, reducing the possibility of failure to respond in a timely manner when a malfunction occurs.

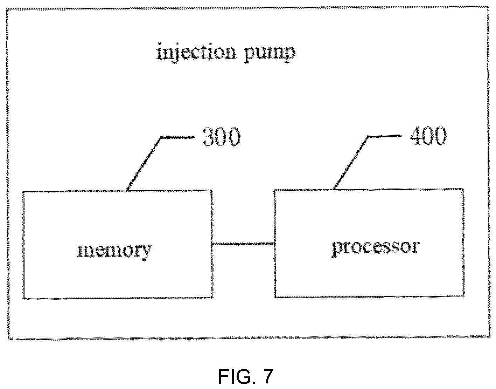

As shown in FIG. 7, an embodiment of the present invention further provides an injection pump that includes a memory 300 and a processor 400. The processor 400 may be the dual controller unit 10 of the injection pump control system of the above embodiments. The storage 300 may be, such as a hard disk drive memory, a non-volatile memory (such as a flash memory or other electronically programmable restricted deletion memory configured to form a solid-state drive), and a volatile memory (for example, a static or dynamic random access memory and the like), and the like, which is not limited in the embodiments of the present invention.

The memory 300 stores executable program codes thereon, and the processor 400 is electrically coupled to the memory 300, calling the executable program codes stored on the memory, and executing the control method of injection pump control system as described above.

Further, an embodiment of the present application further provides a computer-readable storage medium. The computer-readable storage medium may be set in the injection pump of each of the foregoing embodiments, or may be the memory 300 of the embodiment as shown in FIG. 7. A computer program is stored on the computer-readable storage medium, and is executed by the processor to realize the control method of the injection pump control system described in the foregoing embodiments as shown in FIG. 5 and FIG. 6. Further, the computer-storable storage medium may further be a U disk, a mobile hard disk, a read-only memory (ROM), a random-access memory (RAM), a magnetic disk or an optical disk, and other various media that may store the program code.

In the above-mentioned embodiments, descriptions of the embodiments have particular emphasis respectively. For parts that are not described in detail in a certain embodiment, reference may be made to related descriptions of other embodiments.

The above is a description of the injection pump control method, system, and injection pump according to the present invention. For those skilled in the art, according to the ideas of the embodiments of the present invention, changes may be made to specific implementations and application scopes. In summary, the content of this specification should not be construed as a limitation on the present invention.

## Claims

1. A control method of an injection pump control system, comprising:
when a control switch is detected as being connected, a monitoring controller of a dual controller unit turning on a power source to start supplying power;
a master controller of the dual controller unit transmitting communication signals to the monitoring controller, and controlling a data acquisition unit to collect operational data of an injection pump and determining whether the injection pump is operating normally according to the operational data, and controlling an alarm module of the injection pump to alarm when an abnormality is found;
the monitoring controller continuously obtaining communication signals transmitted by the master controller, and cutting off the supply of power of the power source and transmitting an alarm signal when the communication signal is abnormal, the cutoff of the supply of power triggering an emergency source to be turned on to supply power to the alarm module, and the alarm module alarming according to the alarm signal.

2. The control method according to claim 1, wherein the data acquisition unit comprises a pressure detection sensor and an injection position detection device;
controlling the data acquisition unit to collect operational data of the injection pump comprising:
detecting a pressure of a driving device on an injector of the injection pump through the pressure detection sensor, and detecting an injection status of the injector through the injection position detection device.

3. The control method according to claim 1 or 2, wherein the method further comprises:
when it is detected that a master control line of the injection pump is connected to a radio frequency host, the master controller controlling input equipment of the injection pump to stop responding to input operations and execute instructions transmitted by the radio frequency host.

4. The control method according to claim 3, wherein the method further comprises:
when it is detected that a mute button is pressed, the master controller detecting a current output sound signal and correspondingly cutting off an output of the sound signal.

5. The control method according to claim 4, wherein before cutting off the supply of power of the power source and transmitting the alarm signal, the method further comprises:
controlling a motor of the injection pump to gradually stop running according to a preset gradual-stop mode.

6. An injection pump control system comprising:
a dual controller unit, a dual power source unit, a data acquisition unit, a button control unit and alarm module; wherein
the dual controller unit comprises a monitoring controller and a master controller, and the dual power source unit comprises a power source and an emergency source;
the monitoring controller is connected to the master controller, the power source, the button control unit and the alarm module, respectively; the master controller is connected to the data acquisition unit, the emergency source and the alarm module, respectively; the emergency source is connected to the power source and the alarm module, respectively;
the button control unit comprises a control switch, and the monitoring controller is connected to the control switch, the monitoring controller turning on the power source to start supplying power when the control switch is connected;
the master controller is configured to transmit communication signals to the monitoring controller, and controls the data acquisition unit to collect operational data of the injection pump and determines whether the injection pump is operating normally according to the operational data, and controls the alarm module to alarm when an abnormality is found;
the monitoring controller continuously obtains the communication signals transmitted by the master controller, and cuts off the supply of power of the power source and transmits an alarm signal when the communication signal is abnormal, wherein the cutoff of the supply of power triggers the emergency source to be turned on to supply power to the alarm module, and the alarm module alarms according to the alarm signal.

7. The system according to claim 6, wherein the monitoring controller and the master controller are respectively connected to a motor of the injection pump;
the monitoring controller is configured to control the motor to gradually stop running according to a preset gradual-stop mode when the communication signal is abnormal; and
the master controller is configured to control an operating status of the motor according the operational data of the injection pump collected by the data acquisition unit.

8. The system according to claim 6 or 7, wherein the master controller is further connected to a master interface of the injection pump;
the master controller is configured to control input equipment of the injection pump to stop responding to input operations and obtain instructions output by a radio frequency host when it is detected that the master interface is connected to the radio frequency host through a master control line.

9. The system according to claim 8, wherein the button control unit further comprises a mute switch, and the master controller is connected to the mute switch,
when the mute switch is connected, the master controller detects a current output sound signal and correspondingly cuts off an output of the sound signal.

10. An injection pump comprising an injection pump control system as claimed in any one of claims 6-9.
